# EUROPEAN PATENT APPLICATION

(11) **EP 0 626 229 A1**
(43) Date of publication of application: **30.11.1994**
(21) Application number: 94303496.7
(22) Date of filing: 16.05.1994
(51) Int. Cl.: B23K 26/00, H01S 3/16

(54) **Solid state laser for removing physiologic tissue**

(30) Priority: 21.05.1993 US 64456
(71) Applicant: LASER MEDICAL TECHNOLOGY, Inc., San Clemente, California 92673 (US)
(72) Inventor: Levy, Guy, F-83150 Bandol (FR); Danilov, Alexander A., San Clemente, California 92763 (US)
(74) Representative: Spall, Christopher John

(57) **Abstract**

A device for cutting physiologic tissue composed of a handpiece (2) having an output end, an optical system (12,14) carried in the handpiece for conducting laser radiation and for emitting the radiation from the output end; and a laser optically coupled to the optical system for delivering laser radiation to said optical system (12,14). The laser is of a size which permits it to be installed in the handpiece (2). Ideally, the laser constitutes a source of laser radiation at a wavelength substantially between 2.65 and 2.8µm.

## Description

### CROSS-REFERENCE TO RELATED APPLICATION

This is a continuation-in-part of pending application Serial Number 08/025,521 filed on March 3, 1993, and abandoned as of the filing date of the present application.

### BACKGROUND OF THE INVENTION

The present invention relates to medical and dental treatments which involve the use of laser radiation, particularly to remove hard tissue, including bone, enamel, dentin, cementum, and dental root material, and soft tissue, including gum, tooth pulp, and all other vascularized physiologic tissue.

The use of laser radiation to treat various types of biological tissue has been the subject of research over several decades. The research has demonstrated the ability of laser radiation in various wavelength ranges and at various power levels to produce specific effects on selected tissues, including cutting of hard and soft tissues, sterilization, anesthesia or desensitization, hemostasis, etc.

A wide variety of laser systems for performing medical and dental treatments have been developed or proposed.

Some of these systems are constituted by a handpiece containing a laser, as disclosed in U.S. Patent Numbers 3,622,743: 4,503,853; 4,826,431; and 5,074,861. In these devices, the laser may be a semiconductor laser which can produce radiation at a wavelength and power level that do not result in tissue removal but can perhaps serve to perform sterilization or desensitization operations.

Other systems include lasers which operate at power levels that enable more substantial operations to be performed, such as cutting.

By way of example, use has been made of radiation at a wavelength of 10.6 µm produced by a CO₂ laser, 1.06 µm or 0.532 µm produced by an Nd:YAG laser and 2.94 µm produced by an Er:YAG laser. A laser of the last-mentioned type is disclosed in U.S. Patent No. 5,074,861.

It has further been learned that if certain procedures requiring relatively high power levels, such as cutting hard tissue, are performed with pulsed radiation, harmful side effects such as thermal damage to adjacent tissue can be minimized, as disclosed in U.S. Patent No. 5,020,995, which issued to Guy Levy on June 4, 1991, if the laser radiation pulses are applied to the tissue to be cut simultaneously with delivery of a cooling medium containing water, and preferably an air/water spray.

Known lasers capable of generating radiation which can effectively cut biologic tissue operate at very low energy conversion efficiency levels, which means that such a laser must be capable of operating at a very high level of pumping power and must be equipped with a voluminous, high capacity cooling system. Because of these requirements, such lasers must be installed in relatively large floor-mounted housings and a long fiber optic or optical waveguide cable must be used to conduct the radiation to a handpiece or other device which allows properly controlled delivery of the laser radiation.

While fiber optic cables are preferred laser radiation conducting devices, long optical fiber cables are a source of further radiation power losses and long optical fibers which are currently available, such as those made of silica, can not effectively propagate laser radiation at wavelengths longer than 2.5 µm. Therefore, although certain wavelengths longer than 2.5 µm have been considered to be of value for the purposes under consideration, practical systems for delivering such radiation at sufficiently high output power levels have thus far eluded workers in the art.

Known lasers having these capabilities could not, as a practical matter, be installed in a handpiece because these lasers and their cooling system would be unacceptably heavy and bulky.

On the other hand, if a known solid state laser is installed in a handpiece, such as disclosed in U.S. Patent No. 5,074,861, each laser rod can produce only a low level of power because of the extremely low energy conversion efficiency of known solid state laser elements, notably known as Er:YAG laser elements. Any attempt to increase the energy output from such a laser, which could only be achieved by correspondingly increasing the energy input thereto, would inevitably be accompanied by increased heat dissipation from the laser. This would require an increase in the cooling capacity of the laser system and/or a substantial increase in the temperature of the outer surface of the handpiece. As a result, the handpiece would be made too heavy to be easily manipulated and/or would become too hot to be held.

In addition, the device disclosed in U.S. Patent No. 5,074,861 consists of a plurality of laser rods, eight such rods being illustrated in the patent drawing. While these rods are all driven by the same flashlamp, it will inevitably occur, due for example to unavoidable manufacturing imperfections, that the radiation produced by each rod will not be perfectly in phase with the radiation produced by another rod. Therefore, the beam formed by combining the radiation from all rods will be less nonschematic than that emanating from a single rod.

### SUMMARY OF THE INVENTION

It is an object of the present invention to provide a compact, lightweight system for producing pulsed laser radiation capable of safely and effectively cutting hard and soft biologic tissue.

A more specific object of the invention is to provide a pulsed laser which has a relatively high energy conversion efficiency and low lasing energy threshold.

A further specific object of the invention is to provide a laser system which can supply pulsed laser radiation with a high repetition rate capable of effectively cutting hard or soft tissue while requiring minimal cooling of the laser.

Another specific object of the invention is to provide a laser system having the above operating capabilities while being completely contained in a compact, lightweight handpiece.

Yet another specific object of the invention is to provide a system having a short path between the laser rod and the point of treatment.

The above and other objects are achieved by a device for cutting physiologic tissue comprising: a handpiece having an output end; an optical system carried in the handpiece for conducting pulsed laser radiation and for emitting the radiation from the output end; and a solid state laser optically coupled to the optical system for delivering pulsed laser radiation to the optical system, the laser constituting a source of laser radiation at a wavelength substantially between about 2.65 and 2.8 µm. According to preferred embodiments of the invention, the laser includes a lasing medium consisting of a single rod is contained entirely within the handpiece.

### BRIEF DESCRIPTION OF THE DRAWINGS

Figure 1 is a side elevational view illustrating the interior of a handpiece according to a preferred embodiment of the invention.

Figures 2-4 are detail views illustrating specific components of the device shown in Figure 1.

### DESCRIPTION OF THE PREFERRED EMBODIMENTS

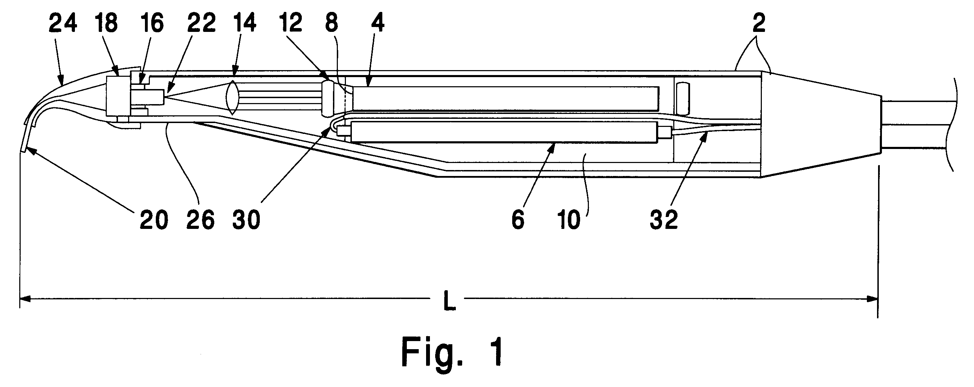

Figure 1 is a side elevational view showing the interior of a handpiece according to the invention. The handpiece is composed of a housing 2 containing all components of a solid state laser, an optical system for outputting a beam of laser radiation and a liquid delivery unit.

The solid state laser is composed of a laser rod 4 provided, in a conventional manner, with reflecting surfaces at its axial ends, a flash lamp 6 which is optically coupled to rod 4 to generate, in accordance with principles well known in the art, laser radiation in rod 4, which radiation is then emitted from the output end 8 of rod 4, and a reflecting enclosure 10 which concentrates light from flash lamp 6 to the maximum achievable extent into rod 4.

The optical system includes a collimating lens 12, a concentrating lens 14, an optical coupler 16, an adapter 18 and an output optical fiber 20.

Collimating lens 12 places the slightly diverging beam from rod 4 into the form of a collimated beam and lens 14 places the collimated beam in the form of a converging beam which is focussed substantially to a point 22.

Coupler 16 supports adapter 18 so as to correctly position adapter 18 relative to lenses 12 and 14, particularly to place the proximal end of fiber 20 at focal point 22. Adapter 18 is of a type which permits ready installation of a replacement fiber 20.

Housing 2 is completed by a boot 24 which surrounds adapter 18 and a portion of fiber 20.

The liquid delivery unit includes a coaxial tube arrangement 26 composed of an interior tube for conducting a water stream and an exterior tube defining, with the interior tube, an annular path for conducting an air stream. The output ends of the tubes are configured to form an air/water spray that will be directed to cause water to flow across material being cut at the location where radiation from fiber 20 impinges on the material. This arrangement may correspond to a conventional dental cooling spray delivery structure, although in this invention water performs a different function, and the intensity of the spray can be controlled by adjusting the water and air flow rates in any conventional manner.

The orientation of fiber 20 may be determined by giving the output end portion of tube arrangement 26 a desired configuration and forming boot 24 to cause fiber 20 to lay next to, i.e. to follow the configuration of, tube arrangement 26. Boot 24 may be of a flexible material, such as rubber, or a thin-walled rigid material.

Rod 4 is a solid body composed of a crystal host material containing activator ions, and possibly sensitizer ions, selected to generate laser radiation at a desired wavelength. By way of example, the host material may be YAlO(3) containing only dispersed activator ions, such as Er³⁺, for example in a proportion of about 5% to 50%, atomic, relative to the host material. According to another example, the host material may be YSGG containing dispersed activator ions, such as Er³⁺, for example in a proportion of about 5% to 50%, atomic, and dispersed sensitizer ions, such as Cr³⁺, for example in a proportion of about 0.5% to about 3%, atomic. This material can produce radiation at a wavelength of 2.78 µm. According to a third example, the host material may be YAG containing ions such as Cr³⁺, Tm³⁺ and Er³⁺. In this third example, Cr³⁺ may be present in a proportion of about 0.5% to about 3%, atomic, Tm³⁺ may be present in a proportion of about 0.3% to about 3%, atomic, and Er³⁺ may be present in a proportion of about 5% to 50%, atomic, all of these ranges being cited by way of preferred examples. A laser according to the third example can be fabricated to produce radiation at a wavelength of the order of 2.69 µm and can produce a radiation beam having a minimum of uncorrectable divergence and which can thus be focussed to a small diameter spot. If one of these exemplary laser rods is excited by inputting to lamp 6 pulses of electrical power having an average value of the order of 500 watts at a repetition rate of not less than 30 pps and a pulse duration of about 0.05 ms to about 0.4 ms, laser radiation having an average power level value of 3-5 watts can be produced at the output end of fiber 20, with passive energy losses, distributed between laser rod 4 and fiber 20, being of the order of 5-20%.

Preferably, fiber 20 is composed of silica, sapphire, CaF, or mixtures thereof.

The cutting of tissue according to the invention is preferably effected by emitting the radiation in a form such that it is concentrated to a small diameter spot at the surface of the tissue to be cut while maintaining, at that surface, a thin water layer which extends across the spot.

Removal of hard and soft tissue according to the invention is effected by a substantially non-thermal mechanical ablation action resulting from interaction of the radiation pulses with the tissue through a thin layer of water. A device according to the invention will act as an essentially non-thermal knife or drill which is most effective if the pulse repetition rate is not less than 30 pps. The action of this device is such that very little heat is transmitted to the tissue being cut. This means that cutting can be performed at a speed which the practicioner will find ideal, with essentially no possibility of burning or carbonization of the tissue being cut.

To effectuate tissue removal, a thin layer of water is provided at the surface of the tissue to be removed and laser radiation is concentrated to a small diameter spot at the water layer. Energy from each radiation pulse is absorbed partly inside the thin later layer and partly by the tissue in a confined region adjacent the water layer. The energy absorbed by the tissue creates a local thermal process which reduces the force required to ablate the tissue. Another part of the energy is absorbed in the thin water layer to create a trapped bubble which then collapses with a speed and intensity that produce an acoustic shock wave that disintegrates, or ablates, the modified tissue. A plurality of bubble-creation/collapse cycles can occur during each radiation pulse. This acoustic mechanism is also described in U.S. Patent No. 5,116,227 which issued to Guy Levy on May 26, 1992. The ablation process is described in greater detail in a copending U.S. application by Guy Levy, filed March 3, 1993, and entitled REMOVAL OF PHYSIOLOGIC TISSUES EMPLOYING VAPOR CAVITATION, and a copending U.S. application by Alexander Danilov, entitled CUTTING MATERIALS WITH LASER RADIATION, the disclosures of which are incorporated herein by reference.

A layer of water is maintained at the surface simply by directing an air/water spray from tube arrangement 26 at the surface being irradiated. In the embodiment of a handpiece according to the invention illustrated herein, the spray is directed substantially coaxially with the axis of the output portion of optical fiber 20.

For supplying operating power to the laser, a power line 30 and a ground line 32 are connected to respective ends of tube 6. These represent the only electrical connections to the handpiece: the only other connection to the handpiece is a conduit for delivering air and water to arrangement 26 and conduits for circulating laser cooling water between the handpiece and a supply tank.

In connection with devices of the type here under consideration, the U.S. FDA may require the provision of a shutter in the path of radiation from the laser. If this should occur, the device according to the invention would additionally be provided with a shutter of conventional design and with four control conductors for controlling and/or monitoring the operating state of the shutter. The conductors would be provided by a thin cable that also constitutes a connection to the handpiece.

The action produced by the laser radiation can be controlled primarily by adjusting the power level, duration and repetition rate of excitation pulses delivered to lamp 6 and secondarily by adjusting the rate of flow of air and water through arrangement 26. All of these parameters can be controlled via a control panel separate from the handpiece or on housing 2. However, it may prove more convenient for the practitioner to provide electric power control circuitry and air and water delivery valves in a control unit and to have only on-off buttons or switches on housing 2.

The thickness of the water layer must be selected to take account of the attenuation, or extinction length, of laser radiation in water. In the wavelength range of 2.65 µm to 2.8 µm, which is more preferred, this extinction depth is about 0.07 mm to 0.1 mm. Three suitable wavelengths in this range are 2.69, 2.71 and 2.78 µm.

In contrast the Er: YAG radiation wavelength has an even shorter extinction length in water, which means that the radiation is so highly absorbed by water that cutting according to the invention is difficult to control. While it is difficult to directly measure the thickness of a water layer being formed by an air/water spray, the intensity of the spray can be made adjustable and can then be easily adjusted by the practitioner to create a water layer which allows the desired cutting action to be achieved. Thus, adjustment of the spray can constitute an auxiliary control of cutting speed.

The cutting mechanism according to the invention can be achieved with liquids other than water. However, water is obviously the preferred liquid in view of its low cost, ready availability and physiologic compatibility.

Preferably, radiation is applied by bringing the distal end of fiber 20 into contact with, or in proximity to, the water film flowing over the tissue to be removed. Under these circumstances, the diameter of the radiation spot will be essentially the same as the diameter of fiber 20. Fiber 20 preferably has a diameter of about 0.2 to 0.5 mm.

If the distal end of fiber 20 is spaced a small distance from the tissue surface, the radiation spot will still have a sufficiently small diameter. However, the cutting action can be controlled most effectively by placing the distal end of fiber 20 in contact with the tissue, or with the water film and then generating radiation pulses to effect cutting.

The radiation employed in the practice of the present invention is in the form of pulses primarily to prevent thermal buildup at the treatment site and secondarily to reduce the average power which must be supplied to the laser while enabling the laser radiation to have the requisite power density. It presently appears that the laser radiation emitted from fiber 20 should have a pulse duration of 0.05 ms to 0.4 ms and a repetition rate not less than 30 pps, and preferably up to 100 pps. As noted earlier, the radiation from fiber 20 can be given an average power level of 3-5 watts with an input power level to the laser system which is sufficiently low to assure that the laser rod will not experience thermal destruction or damage and that the housing will not become uncomfortably warm. A handpiece according to the invention may be made to have a length, L, of less than 50 cm, and preferably as small as 19 cm. The weight of the handpiece can be made as low as 100 g.

While there are known laser devices which emit radiation capable of cutting hard and soft tissue, all such known devices have a very low global energy conversion efficiency which requires a high level of pumping power, i.e. energy delivery rate, and thus the provision of a substantial water cooling system for the laser, as described earlier herein.

The pumping energy, i.e. the energy supplied to a flash lamp, has two components: that required to reach the lasing threshold of the lasing medium; and that which is converted to laser radiation.

As an example of a known lasing medium, a typical Er:YAG laser has a lasing threshold of 25-35 J, which must be supplied for generating each lasing pulse. Thus, for a pulse repetition rate of 30 pps, an average pumping power of 750 W-1050 W is expended just to reach the lasing threshold. Such a laser typically has an energy conversion efficiency, taking into account losses in a typical optical fiber delivery system, of the order of about 1%. Therefore, to produce laser radiation at an average power level of 5W, an additional average pumping power component of 500 W must be supplied to the flash lamp. Therefore, the total average power supplied to the flash lamp will be between 1250 and 1550 W, hence the need for substantial cooling.

In contrast, the lasing media according to the present invention have a lasing threshold of about 2 J per pulse for operation at a pulse rate of the order of 30 pps, corresponding to an average pumping power of 60 W to reach the lasing threshold. In addition, the lasing media according to the invention have an energy conversion efficiency of the order of about 2%, due in part to the possibility of using a short optical fiber delivery system. Therefore, to produce laser radiation having an average power of 5 W, an average pumping power component of 250 W is required, resulting in a total average pumping power level of only about 300 W, or about 20% of that required for a typical known laser producing the same laser radiation power level.

As a result, the cooling system for a laser according to the invention can have a substantially lower heat dissipating capacity, and thus a substantially smaller size, than that of conventional lasers, which means that the requisite cooling system can be built into a handpiece of acceptable size. Based on calculations, a handpiece according to the invention may operate satisfactorily with a cooling water flow of the order of about 1 liter/min, with a separately housed water supply tank having a capacity of about 2 liters and equipped with a pump. At a maximum pumping power level of 300 W, and an ambient temperature of 20° C, the laser handpiece will be maintained at a temperature of no greater than 29° C.

In view of the low level of cooling water flow required by the handpiece, the tubes for circulating cooling water between the handpiece and the supply tank can have relatively small diameters and the pump associated with the supply tank can be relatively small. Within the handpiece, cooling water circulating systems can be designed according to principles well known in the relevant art.

Moreover, the laser wavelengths according to the present invention, when applied in the presence of a water film, exhibit a surprisingly low cutting threshold even for cutting healthy enamel, dentin, or cementum. For example, if radiation having a wavelength of 2.78 µ in the form of pulses having a duration of 160 µsec and a repetition rate of 30-50 pps is delivered via a fiber having a diameter of 350 µm and having its distal end in contact with the tissue or the water film, cutting will begin to be observed at an output energy level of 1.6 mJ per pulse.

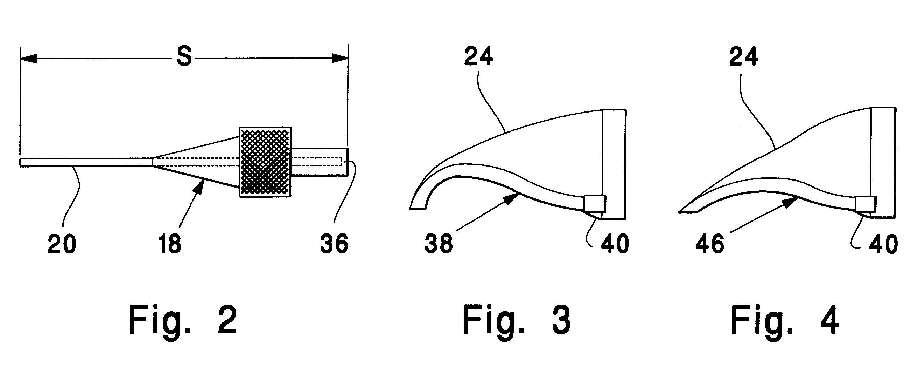

Figure 2 is a detail view of adapter 18 of Figure 1. The adapter illustrated is an SMA adapter, which is an industry standard optical fiber holder. Fiber 20 is shown in an extended, or linear condition, in which case the overall length S of adapter 18 and fiber 20 may be of the order of 5 cm. Fiber 20 is held in adapter 18, and adapter 18 is positioned in coupler 16, such that the center of the proximal end 36 of fiber 20 coincides as closely as possible with focal point 22.

Figure 3 is a detail view of boot 24 with an outlet portion 38 of coaxial tube arrangement 26. Boot 24 is configured to direct the distal end of fiber 20 at an angle of about 70° to the longitudinal axis of the handpiece. If the practitioner desires a different orientation for the output end of fiber 20, boot 24 outlet portion 38 may be replaced. For this purpose, outlet portion 38 includes a coupler 40 for effecting a plug-in connection with the remainder of arrangement 26.

Figure 4 shows a boot 24 for holding the distal end of fiber 20 at a smaller angle, e.g. 35°, to the longitudinal axis of the handpiece. Here, arrangement 26 includes an outlet portion 46 configured to give fiber 20 the desired orientation.

In the illustrated embodiment, boot 24 is flexible and at least slightly elastically stretchable and has a small diameter open end which fits around the exit end of portion 46. When a boot is installed on housing 2, fiber 20 will slide through the small diameter open end of the boot and will then be elastically pressed by the boot against outlet portion 38 or 46. Thus, the shape of the outlet portion determines the orientation of the distal end of fiber 20 relative to the longitudinal axis of the handpiece.

While the description above refers to particular embodiments of the present invention, it will be understood that many modifications may be made without departing from the spirit thereof. The accompanying claims are intended to cover such modifications as would fall within the true scope and spirit of the present invention.

The presently disclosed embodiments are therefore to be considered in all respects as illustrative and not restrictive, the scope of the invention being indicated by the appended claims, rather than the foregoing description, and all changes which come within the meaning and range of equivalency of the claims are therefore intended to be embraced therein.

## Claims

1. A device for cutting physiologic tissue comprising: a handpiece having an output end; an optical system carried in said handpiece for conducting laser radiation and for emitting the radiation from the output end: and a solid state laser optically coupled to said optical system for delivering laser radiation to said optical system, said laser constituting a source of laser radiation at a wavelength substantially between 2.65 and 2.8 µm.

2. A device as defined in claim 1 wherein said laser comprises a lasing medium consisting of a single rod of lasing material.

3. A device as defined in claim 2 wherein said laser is contained entirely within said handpiece.

4. A device as defined in claim 3 wherein said optical system comprises an optical fiber having a distal end projecting from the said output end of said housing, and a focussing system disposed for concentrating light from said laser into said optical fiber.

5. A device as defined in claim 4 wherein said laser comprises a laser rod in which the laser radiation is generated, said laser rod having length dimension and a longitudinal axis extending along the length dimension and along which laser radiation is emitted from said rod, said focussing system has an optical axis aligned with said longitudinal axis of said rod, and the sum of the length of said rod and the distance between said rod and the distal end of said optical fiber is not greater than 50 cm.

6. A device as defined in claim 5 wherein said rod comprises a host material containing Er³⁺ activator ions.

7. A device as defined in claim 6 wherein the host material is YAlO with a content of about 5% to about 50%, atomic, Er³⁺.

8. A device as defined in claim 6 wherein the rod further comprises Cr³⁺ sensitizer ions.

9. A device as defined in claim 8 wherein the host material is YSGG with a content of about 0.5% to about 3%, atomic, Cr³⁺.

10. A device as defined in claim 9 wherein the host material has a content of about 5% to about 50%, atomic, Er³⁺.

11. A device as defined in claim 6 wherein the host material is YAG with a content of about 5% to about 50%, atomic, Er³⁺, about 0.5% to about 3%, atomic, Cr³⁺ and about 0.3% to about 3%, atomic, Tm³⁺.

12. A device as defined in claim 5 further comprising conduit means carried by said handpiece for delivering a liquid spray from said output end of said handpiece in the direction of the tissue to be cut.

13. A device as defined in claim 5 wherein said handpiece has a maximum dimension of not more than 30 cm.

14. A device as defined in claim 13 wherein the maximum dimension of said handpiece is not more than 20 cm.

15. A device as defined in claim 5 wherein said handpiece has an elongate tubular form.

16. A device as defined in claim 3 wherein the laser radiation wavelength is 2.69 µm, 2.71 µm or 2.78 µm.

17. A device as defined in claim 1 in combination with an electric power source and an electric cable connected for delivering power from said source to said laser.

18. A device as defined in claim 1 wherein said laser is a cooled device which supplies radiation at an average power of 3-5 watts at said output end of said handpiece.
